# EUROPÄISCHE PATENTSCHRIFT

(11) **EP 1 409 031 B1**
(45) Veröffentlichungstag und Bekanntmachung des Hinweises auf die Patenterteilung: **27.04.2005**
(21) Anmeldenummer: 02754867.6
(22) Anmeldetag: 11.07.2002
(51) Int. Cl.: A61L 15/44

(54) **BLUTSTILLENDES HAUTPFLASTER**
HEMOSTATIC SKIN ADHESIVE DRESSING
PANSEMENT HEMOSTATIQUE ADHESIF CUTANE

(30) Priorität: 20.07.2001 DE 10135507
(43) Veröffentlichungstag der Anmeldung: 21.04.2004
(73) Patentinhaber: Henkel Kommanditgesellschaft auf Aktien, 40589 Düsseldorf (DE)
(72) Erfinder: KREMER, Joachim, 40764 Langenfeld (DE); FERENCZ, Andreas, 40223 Düsseldorf (DE); HUVER, Thomas, 40625 Düsseldorf (DE); GAWRISCH, Wolfgang, 40589 Düsseldorf (DE)
(86) Internationale Anmeldenummer: PCT/EP2002/007738
(87) Internationale Veröffentlichungsnummer: WO 2003/011172

(56) Entgegenhaltungen:
- DE-A- 3 315 678
- DE-B- 1 190 608
- US-A- 3 328 259
- US-A- 3 987 000
- US-A- 4 022 203
- US-A- 4 395 398
- US-A- 4 990 339
- US-A- 5 785 955

## Beschreibung

Die Erfindung betrifft ein Blutstillendes Hautpflaster mit diskreter Form, dadurch gekennzeichnet, dass das Pflaster einen einschichtigen Polymerfilm mit mindestens einem darin gelösten oder dispergierten, adstringierenden und/oder blutstillenden Wirkstoff umfasst, wobei einer der adstringierenden und/oder blutstillenden Wirkstoffe ausgewählt ist aus Tanninen, Aluminium-, Zink-, Zirkonium-, Calcium- oder Bismutsalzen.

Kleine Schnitte oder Kratzer können z.B. im Gesicht oder an den Händen auch bei oberflächlicher Verletzung der Haut starke Blutungen verursachen. Pflaster, die solche Wunden abdecken sollen, sind im allgemeinen mit einer Wundauflage versehen, die das Blut und die Wundsekrete aufnehmen kann. Diese wird mit einer zweiten, klebenden Schicht über der Wunde befestigt.

Nachteilig ist bei solchen handelsüblichen Wundschnellverbänden, hier im weiteren als Pflaster bezeichnet, dass die Wunde häufig mit der Wundauflage durch das ausgetretene Blut verklebt. Dadurch kann die Wunde beim Abnehmen des Pflasters wieder aufreißen und so neue Blutungen und Schmerzen verursachen.
Zudem bedecken solche Pflaster einen im Vergleich zur Wunde deutlich größeren Hautbereich, da sie durch Klebeflächen fixiert werden müssen. Dies führt dazu, dass sich Verletzungen an exponierter Stelle, z.B. im Gesicht, nicht unauffällig versorgen lassen.
Bei ungeeigneter Form oder Größe der herkömmlichen Klebepflaster kann die Klebefläche außerdem mit dem Rand der Wunde in Kontakt kommen. Neben einer Verschmutzung der Wunde durch die Klebstoffe ist eine häufige Folge davon ein mit Schmerzen verbundenes Aufreißen der Wunde beim Entfernen des Pflasters.

Die in solchen Pflastern verwendeten Klebstoffe können außerdem Hautirritationen oder Allergien auslösen und hinterlassen oftmals beim Entfernen Klebstoffreste auf der Haut, die mit organischen Lösungsmitteln entfernt werden müssen. Zur Abdeckung von kleinen Wunden, mit eher geringer Oberfläche, z.B. bei Kratzern oder Schnitten, sind diese Pflaster daher ungeeignet.

Die Patentschrift US 4,233,976 offenbart ein poröses Netz aus hydrophoben oder wasserabweisenden Fasern von mindestens 50 µm Dicke beschichtet mit 0,05 bis 0,9 g pro cm³ Netz einer adstringierenden Substanz in kristalliner Form. Nachteilig ist beispielsweise, dass Blut durch das Netz fließt und so unter anderem Kleider verschmutzen kann.

Bei Sprühverbänden werden Polymere in flüchtigen organischen Lösungsmitteln gelöst und auf die Haut aufgesprüht (z.B. US 3,073,794 oder US 3,476,853). Nach dem Verdampfen der Lösungsmittel bleibt auf der Haut ein Polymerfilm zurück, der eine uneinheitliche, besonders in den Randbereichen verringerte Schichtdicke aufweist. Solche Sprühverbände können, wie z.B. in US 3,987,000 beschrieben, auch Therapeutika und andere Wirkstoffe enthalten.
Nachteilig bei Sprühverbänden ist es, dass die verwendeten organischen Lösungsmittel auf der Haut und in der Wunde Brennen und Hautirritationen verursachen können. Zu dem lässt sich der Polymerfilm an den dünnen Ränder kaum so fassen, dass er restlos abgezogen werden kann. Zur vollständigen Entfernung des Sprühverbands muss die Haut daher mit Wasser abgewaschen werden. Dieses Abwaschen führt aber häufig dazu, dass die Wunde wieder aufbricht, wobei Keime in die Wunde gelangen können.
Das Auftragen der Sprühverbände auf Wunden ist nicht punktgenau möglich, wodurch unnötigerweise eine deutlich größere Hautfläche im Vergleich zur Wunde durch das Pflaster bedeckt wird. Andererseits kann es auch partiell aufgrund der Ungleichmäßigkeit der Applikation zu einer mangelhaften Abdeckung von Wunden kommen. Darüber hinaus kann durch die Ungenauigkeiten beim Sprühen z.B. die Kleidung verunreinigt werden. Für die Verwendung bei kleinen Wunden bieten sich die Sprühverbände daher nicht an.

Ein Verfahren zur Herstellung eines zweischichtigen Wundverbandes wird in DE 11 90 608 beschrieben. Eine aus thermoplastischen Kunststoffen bestehende Folie, die für den Austritt von Blut und Sekreten durch Schnitte oder Einstiche perforiert ist, wird wundseitig mit einem filmbildenden, wasserlöslichen Kunststoff beschichtet, dem u.a. Bakterizide, extraktive und/oder hämostatische Substanzen zugesetzt sind, und an der Wunde abgekehrten Seite mit über der Folie angebrachten Lagen saugfähigen Materials verbunden oder bedeckt. Durch die Perforation treten Blut und Wundsekrete aus und werden von dem saugfähigen Material aufgenommen. Dieser Verband dient vor allem zur Beobachtung und Versorgung größerer Wunden und muss zudem mit Pflastern oder anderen Klebevorrichtungen fixiert werden.
Aufgrund seines komplexen Aufbaus ist das in DE 11 90 608 beschriebene Pflaster für die einfache und unauffällige Anwendung bei der Versorgung vor allem kleiner Wunden nicht geeignet.

In der Patentschrift US 3,328,259 wird ein Verband offenbart, der einen flexiblen Körper umfasst, wobei der Körper ein wasser- und plasmalösliches Cellulosederivat beinhaltet, das hämostatische und filmbildende Eigenschaften aufweist und sich mit dem Plasma in der Wunde zu einer künstlichen wasserunlöslichen Kruste verbindet.
Nachteilig an diesem Verband ist, dass er nicht einfach wieder zu entfernen ist. Der Verband verklebt selbst mit der Wunde und bildet eine künstliche Kruste aus, die das einfache Entfernen des Verbands (insbesondere durch Abziehen) ohne ein Wiederaufreißen der Wunde unmöglich macht. Bei kleinen Wunden ist aber eine besonders wünschenswerte Eigenschaft, dass der Verband ohne weitere Hilfsmittel, wie z.B. organische Lösungsmittel oder Wasser, wieder entfernt werden kann.

Aufgabe der Erfindung ist es daher, ein leicht handhabbares, punktgenau anzuwendendes und einfach wieder zu entfernendes Pflaster mit guter blutstillender Wirkung bereitzustellen, das besonders für kleine Wunden geeignet ist.

Diese Aufgabe wird gelöst durch ein blutstillendes Hautpflaster mit diskreter Form, das dadurch gekennzeichnet ist, dass es einen einschichtigen Polymerfilm mit mindestens einem darin gelösten oder dispergierten, adstringierenden und/oder blutstillenden Wirkstoff umfasst, wobei einer der adstringierenden und/oder blutstillenden Wirkstoffe ausgewählt ist aus Tanninen, Aluminium-, Zink-, Zirkonium-, Calcium- oder Bismutsalzen.

Das erfindungsgemäße Hautpflaster weist eine diskrete Form auf. Unter diskreter Form ist in dem erfindungsgemäßen Zusammenhang zu verstehen, dass das Pflaster definierte Randbereiche hat. Die Schichtdicke im gesamten Hautpflaster ist im wesentlichen gleich und wird nicht zu den Rändern hin dünner bzw. unregelmäßiger, wie es bei den Sprühpflastern meist vorkommt.
Es sind aber auch Pflaster denkbar, die für spezielle Anwendungen ein Profil aufweisen. Die verschiedenen Bereiche des Profils besitzen dabei unterschiedliche, definierte Schichtdicken. Ein einfaches Profil könnte beispielsweise eine geringe, im wesentlichen gleichmäßige Schichtdicke über der Wunde aufweisen, wodurch z.B. die Luftdurchlässigkeit des Pflasters erhöht sein kann, während die im Vergleich dazu dickeren Ränder das Ablösen des Pflasters vereinfachen.

Eine Möglichkeit zur Herstellung der diskreten Form ist beispielsweise das Ausstanzen des erfindungsgemäßen Pflasters aus einem entsprechenden Polymerfilm. Die dreidimensionale Form (z.B. Länge, Breite und Dicke) kann dabei für bestimmte Anwendungen optimiert sein, beispielsweise als rundes Pflaster, besonders geeignet für Rasurverletzungen im Gesicht, oder rechteckig für längere Schnitte. Auch kompliziertere, beispielsweise auf bestimmte Hautstellen (wie Fingerkuppen) angepasste, vorgegebene Formen sind denkbar.
Die diskrete Form kann aber auch vom Anwender selbst erzeugt werden, indem er das Pflaster durch z.B. Aus- oder Abschneiden an die Erfordernisse der Verletzung angepasst. Entsprechende Darreichungsformen des Pflasters sind beispielsweise als Folie (z.B. in der Größe DIN A5) oder als Endlosrolle denkbar.

Der Polymerfilm des erfindungsgemäße Pflaster ist insbesondere nach Befeuchten mit wässrigen Flüssigkeiten, wie z.B. Wasser, Blut oder Wundsekret, selbstklebend.
Aufgrund dieser Eigenschaft hat das erfindungsgemäße Pflaster nahezu vollflächigen mechanischen Kontakt mit der Haut und haftet auch direkt auf der Wunde. Dadurch wird keine weitere Klebevorrichtung zur Befestigung des Hautpflasters benötigt.

Deshalb kann im Gegensatz zu herkömmlichen, häufig in größeren Stücken oder als Endlosrollen angebotenen Pflastern, die i.d.R. nur an zwei Seiten vorhandenen Klebeschichten besitzen, die Größe des erfindungsgemäßen Pflaster deutlich geringer sein. Das Pflaster selbst ist daher schon wegen der geringeren Größe unauffälliger. Die Form ist dabei frei wählbar und kann so individuell auf die Verletzung zugeschnitten werden, ohne dass mit einer schlechteren Haftung gerechnet werden muss.

Das erfindungsgemäße Hautpflaster ist flexibel und passt sich durch die verbesserte Haftung im gesamten Wundbereich auch auf vielbewegten Hautpartien, wie z.B. Gesicht oder Händen, den Konturen sehr gut an, ohne ein Spannungsgefühl zu erzeugen.

Trotz der guten Klebeeigenschaften des Polymers lässt sich das erfindungsgemäße Pflaster einfach nach Gebrauch am Rand fassen und direkt, ohne Schmerzen von der Haut abziehen. Zudem bleiben keine Klebstoffreste auf der Haut zurück, die durch organische Lösungsmittel entfernt werden müssen. Es entfällt ebenfalls das Abwaschen des Pflasters mit Wasser, wie es bei Sprühverbänden notwendig ist. Ein Aufbrechen und eine sich möglicherweise anschließende Kontamination der Wunde mit Keimen kann so vermieden werden.

Da keine zusätzlichen Klebevorrichtungen aufgrund der Polymereigenschaften notwendig sind, können mögliche Allergien und/oder Hautirritationen aufgrund einer bestehenden Unverträglichkeit gegen die herkömmlichen Klebstoffe in handelsüblichen Pflastern, im allgemeinen Haftschmelzkleber, einfach vermieden werden.
Es bietet zudem durch die bessere Abdeckung der Wunde effektiveren Schutz vor Bakterien und Schmutz sowie vor Verunreinigung der Kleidung durch Blut oder Wundsekrete.

Der Polymerfilm des erfindungsgemäßen Pflasters kann Poren aufweisen, wobei dann die Porengröße maximal 5 µm beträgt. Diese Poren besitzen einen so geringen Durchmesser, dass das erfindungsgemäße Pflaster keine makroskopisch sichtbaren Perforationen aufweist, durch die beispielweise Wundsekret oder Blut durch das Pflaster hindurchtreten und z.B. die Kleidung verschmutzen könnte. Trotzdem können durch die Poren Schädigungen der Haut aufgrund von Luftabschluss vermieden werden. Perforationen werden allenfalls zum Zwecke der Bereitstellung des Pflasters wie beispielweise zum Herauslösen von vorgestanzten Pflasterformen aus einer Polymerfolie verwendet.

Der Polymerfilm umfasst mindestens einen darin gelösten oder dispergierten, adstringierenden und/oder blutstillenden Wirkstoff. Dieser Wirkstoff gelangt bei der Anwendung des erfindungsgemäßen Hautpflasters in Kontakt mit der Wunde und führt aufgrund seiner adstringierenden und/oder blutstillenden Eigenschaften innerhalb weniger Minuten zu Blutungsstillstand und Wundverschluss. Das Pflaster verklebt daher nicht mit der Wunde selbst und es werden auch keine sichtbaren Koagulate gebildet, die ihrerseits mit dem Pflaster verkleben könnten. Die Wunde bricht so beim Abziehen des Films nicht mehr auf und erneutes Bluten wird damit vermieden. So ermöglicht der adstringierende und/oder blutstillende Wirkstoff insbesondere auch die einfache Wiederentfernbarkeit des erfindungsgemäßen Pflasters.

Erfindungsgemäß verwendbare Adstringenzien sind unter anderem Salze mit zwei- und höherwertige Kationen, beispielsweise die Kationen des Zinks, Bismuts, Calciumsund Zirkoniums, sowie die dreiwertigen Kationen des Aluminiums.

Das erfindungsgemäße Hautpflaster ist einfach handhabbar, unauffällig auch an exponierter Stelle zu tragen, gut haftend und punktgenau anzuwenden. Es führt schnell zum Blutungsstillstand und kann einfach und ohne Anwendung von Lösungsmitteln oder Wasser wieder entfernt werden. Das Pflaster vereinbart insgesamt eine einfache, optisch unauffällige Anwendung mit einer hervorragenden medizinischen Wirkung bei der Wundbehandlung.

Gemäß einer besonderen Ausführungsform umfasst der Polymerfilm des erfindungsgemäßen Pflasters ein oder mehrere Polymere. Die Eigenschaften des Polymerfilms können durch die entsprechende Wahl der Molekulargewichte und durch Mischungen von mehreren Polymeren verbessert werden. Geeignet sind dabei vor allem solche Polymere, die wasserlöslich oder mit Wasser quellend sind.

Nach einer besonders bevorzugten Ausführungsform sind das Polymer bzw. die Polymere ausgewählt aus der Klasse der Polyvinylalkohole, Polyvinylpyrrolidone, Vinylpyrrolidon-Vinylacetat-Copolymere, Polysaccharide, Polypeptide, Polyoxazoline, Homo- und Copolymerisate der Vinylphosphon-, Acryl- oder Methacrylsäure oder deren Derivate, beispielsweise deren Ester und Amide, wie z.B. Acrylsäure-ethyl-hexylester oder Acrylamido-methyl-propan-sulfonsäure. Insbesondere sind Polyvinylalkohole oder Polyvinylpyrrolidone geeignet.

Unter Polysacchariden im erfindungsgemäßen Zusammenhang sind beispielsweise Alginsäure oder ihre Salze (z.B. Natrium- oder Calciumalginat), Dextran, Stärke oder Stärkeether, Cellulose oder Celluloseether (wie z.B. Methylcellulose, Hydroxyethylcellulose, Hydroxyethylmethylcellulose, Hydroxypropylcellulose, Hydroxypropylmethylcellulose, Carboxymethylcellulose (z.B. als Natrium- oder Kaliumsalz)) oder andere Cellulosederivate (wie z.B. Oxycellulose, oder Celluloseester wie Cellulosebutyrat, Celluloseacetobutyrat oder Celluloseacetat), Mannane (z.B. Galactomannane oder Galactomannanderivate), Pflanzengummen (u.a. Xanthan oder Gummi Arabicum), Chitin oder Chitosane, Agarose oder Agar Agar zu verstehen. Aus dieser Gruppe eignen sich besonders Alginate, Dextran, Chitosane oder Ether von Stärke oder Cellulose, von diesen insbesondere Carboxymethylcellulose, Hydroxyethylcellulose, Hydroxypropylcellulose oder Hydroxypropylmethylcellulose. Ganz besonders bevorzugt für das erfindungsgemäße Hautpflaster geeignet ist Hydroxyethylcellulose.

Erfindungsgemäße Polymere ausgewählt aus der Gruppe der Polypeptide sind z.B. Kollagen, Caseine oder Gelatine.

Auch Polyvinylphosphonsäure sowie deren Natrium- oder Kaliumsalze, Polyacrylate wie z.B. Methacrylate, Acrylate oder deren Derivate, Copolymere von Vinylphosphonsäure, Acrylsäure, Methacrylsäure oder Derivaten dieser Monomere, wie z.B. Ester oder Amide, miteinander oder anderen geeigneten Substanzen, Polyethylenoxide, Polypropylenglykole oder Vinylpyrrolidon-Vinylacetat-Copolymere können zur Herstellung des erfindungsgemäßen Pflasters verwendet werden.
Als weitere Materialien für das erfindungsgemäße Pflaster sind auch Polyoxazoline (auch als Poly(2-oxazoline) oder Poly(N-acylaziridine) bezeichnet) oder Blockpolymere von hydrophilen Polyurethanen mit Polyethylenglykol geeignet.

Der Vorteil dieser besonderen Ausführungsform des erfindungsgemäßen Hautpflasters besteht darin, dass der aus diesen Polymeren hergestellte Film durch Benetzung mit wässrigen Lösungen, wie z.B. Wasser, Blut oder Wundsekrete, angelöst wird. Der Polymerfilm wird dadurch besonders klebrig und die adstringierenden und/oder blutstillenden Wirkstoffe, die darin gelöst oder dispergiert sind, können mit der Wunde in Kontakt treten. So wird die Blutstillung und das Schließen der Wunde beschleunigt.
Das Anlösen des Polymerfilms findet auf der Wunde aber nur oberflächlich statt, da der adstringierende Wirkstoff die Blutung sehr rasch zum Stillstand bringt und so ein übermäßiges Auflösen des Films vermieden wird. Dadurch besitzt der Film nach der Anwendung noch ausreichende Dicke und mechanische Stabilität, so dass er problemlos von der nun verschlossenen Wunde abgezogen werden kann.

Die Polymere können im erfindungsgemäßen Hautpflaster außerdem auch vernetzt vorliegen. Bei Vernetzungsgraden zwischen ca. 0,1 und 2 % ist die Klebrigkeit des Polymerfilms noch ausreichend, um eine gute Haftung auf der Haut zu gewährleisten, während gleichzeitig die Kohäsion und damit die Wiederentfernbarkeit des Hautpflasters verbessert wird. Eine entsprechende Vernetzung kann in dem Fachmann bekannter Weise z.B. bei Polyvinylalkoholen durch die Reaktion mit Glyoxal, Metallsalzen (beispielsweise Titan(IV)-salzen) oder Borsäure herbeigeführt werden.

Eine weitere bevorzugte Ausführungsform des erfindungsgemäßen Pflasters ist dadurch gekennzeichnet, dass das Pflaster weitgehend transparent oder wenig opak ist. Besonders bevorzugter Weise ist das erfindungsgemäße Pflaster farblos. Dies ist z.B. beim Einsatz von Aluminiumsalzen gegeben.
Ein transparentes Pflaster ist unauffälliger als die üblichen Heftpflaster und kann deshalb auch auf exponierten Hautstellen, z.B. im Gesicht, angewendet werden, ohne zu stören. Es ist aber auch möglich, dass Pflaster leicht hautfarben einzufärben, um die Unauffälligkeit weiter zu verbessern.
Das transparente Pflaster hat außerdem den Vorteil, dass die Wunde beobachtet und der Zeitpunkt, an dem die Blutstillung und/oder der Verschluss der Wunde eintritt, besser bestimmt werden kann, als dies bei herkömmlichen, nicht transparenten Pflastern möglich ist. Das Ende der Behandlung ist so einfacher abzuschätzen.

Gemäß einer besonders bevorzugten Ausführungsform des erfindungsgemäßen Pflasters weist der Polymerfilm eine Schichtdicke zwischen etwa 0,001 und 1 mm auf. Nach einer besonders bevorzugten Ausführungsform beträgt die Schichtdicke des Pflasters etwa 0,01 bis 0,6 mm, insbesondere etwa 0,01 bis 0,25 mm. Dies ermöglicht eine gute Handhabbarkeit bei der Applikation sowie beim Entfernen des Pflasters. Außerdem wird gewährleistet, dass das Pflaster sich während der Anwendung nicht in den Wundsekreten oder dem Blut vollständig auflöst. Auch Transparenz und Luftdurchlässigkeit des Pflasters werden durch die geringen Schichtdicken begünstigt.

Der Feuchtigkeitsgehalt des Polymerfilms im erfindungsgemäßen Hautpflaster liegt üblicherweise zwischen 0 und 50 Gew.-%. In einer besonders bevorzugten Ausführungsform beträgt der Feuchtigkeitsgehalt des Polymerfilms zwischen ca. 10 und 35 Gew.-%. Dadurch ist die Applikation des erfindungsgemäßen Pflasters zur Abdeckung von Wunden auch möglich, wenn zum Befeuchten des Pflasters beispielsweise nur keimbelastetes Wasser verfügbar ist. Vemetzte Polymerfilmen können im Vergleich zu unvernetzten Polymeren noch deutlich mehr Wasser aufnehmen, ohne dass die Kohäsion des Pflasters und damit seine Funktionalität entscheidend beeinträchtigt wird.
Die Darreichungsform als feuchtes Pflaster kann z.B. in einer Verpackung aus feuchtigkeitsundurchlässigem Material erfolgen.

Einer der erfindungsgemäß verwendeten, adstringierenden und/oder blutstillenden Wirkstoffe ist ausgewählt aus der Gruppe der Tannine, Aluminium-, Zink-, Zirkonium-, Calcium- oder Bismutsalze. Diese Substanzen lösen sich gut in der Wunde und führen durch ihre adstringierende Wirkung zu einem schnellen Wundverschluss.

Geeignete Calciumsalze sind beispielsweise die Lactate oder Panthotenate; die Zinksalze können u.a. als Lactate, Tannate, Phenolsulfate, Salicylate, Nitrate, Sulfate, Bromide oder Acetate vorliegen.
Ebenfalls können Tannine, auch als Gallotannine bezeichnet, und insbesondere das Tannin (auch als Gallusgerbsäure, Gerbsäure oder Tanninsäure bezeichnet) sowie das Tannin-Albuminat als adstringierende Komponenten eingesetzt werden. Tannine und insbesondere das Tannin können aus verschiedenen pflanzlichen Materialien, wie z.B. Eichenrinde oder Rosskastanie, in dem Fachmann bekannter Weise gewonnen werden.
Gemäß einer besonders bevorzugten Ausführungsform der Erfindung sind die Aluminiumsalze ausgewählt aus Aluminium-Hydroxychloriden (z.B. AluminiumChlorhydrat), Aluminiumderivaten von Allantoin (insbesondere Aluminium-Chloro-Hydroxy-Allantoinat oder Aluminium-Dihydroxy-Allantoinat), Kalium-Aluminiumsulfat, Ammonium-Aluminiumsulfat, Aluminium-Zirkonium-Hydroxychloriden (z.B. Aluminium-Zirkonium-Pentachlorohydrat) oder Aluminium-Zirkonium-Hydroxychlorid-Glycin-Komplex.

Nach einer weiteren bevorzugten Ausführungsform der Erfindung enthält der Polymerfilm Glycerin, Glykole, Sorbitol und/oder andere geeignete Substanzen, die dazu dienen, das Pflaster flexibler und geschmeidiger zu machen, und so die Anpassung an die Hautoberfläche zu verbessern. Besonders geeignete Glykole sind Glycerin, 1,2-Propylenglykol oder 1,3-Butenylglykol, besonders bevorzugt ist Glycerin. Durch diesen Zusatz wird zudem das Auskristallisieren von adstringierenden Salzen im Polymerfilm verhindert.

Gemäß einer besonders bevorzugten Ausführungsform des erfindungsgemäßen Pflasters sind ein oder mehrere Wirk-, Pflege- und/oder Hilfsstoffe enthalten. Darunter sind Haut-Feuchthaltemittel (wie z.B. Pyroglutaminsäure), Vitamine, Antioxidantien, Desinfektionsmittel, regenerative Substanzen oder entzündungshemmende Mittel, Salze, Pflanzenextrakte (wie z.B. insbesondere feuchtigkeitsspendende Substanzen aus Aloe Vera oder entzündungshemmende Kamilleextrakte), Harnstoff, Albumin, Proteinhydrolysate, Aminosäuren, Emulgatoren oder Fette, Öle oder Wachse (z.B. Paraffine oder pflanzliche Fette, Öle oder Wachse) zu verstehen. Auch Substanzen zur Einstellung eines geeigneten pH-Wertes (vorzugsweise im hautneutralen pH-Bereich) sind unter diese Hilfsstoffe zu fassen. Emulgatoren können als oberflächenaktive Substanzen dazu dienen, die verwendeten Substanzen im erfindungsgemäßen Pflaster zu emulgieren oder zu dispergieren. Geeignete Emulgatoren sind beispielsweise:
- Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole,
- C₁₂-C₂₂-Fettsäuremono- und -diester von Anlagerungsprodukten von 1 bis 30 Mol Ethylenoxid an C₃-C₆-Polyole, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen, z. B. das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten C₈-C₂₂-Fettsäuren,
- Sterole (Sterine). Als Sterole wird eine Gruppe von Steroiden verstanden, die am C-Atom 3 des Steroid-Gerüstes eine Hydroxylgruppe tragen und sowohl aus tierischem Gewebe (Zoosterole) wie auch aus pflanzlichen Fetten (Phytosterole) isoliert werden. Beispiele für Zoosterole sind das Cholesterol und das Lanosterol. Beispiele geeigneter Phytosterole sind Beta-Sitosterol, Stigmasterol, Campesterol und Ergosterol. Auch aus Pilzen und Hefen werden Sterole, die sogenannten Mykosterole, isoliert.
- Phospholipide, vor allem die Glucose-Phospolipide, die z. B. als Lecithine bzw. Phosphatidylcholine aus z. B. Eidotter oder Pflanzensamen (z. B. Sojabohnen) gewonnen werden,
- Fettsäureester von Zuckern und Zuckeralkoholen wie Sorbit,
- Polyglycerine und Polyglycerinderivate, bevorzugt Polyglyceryl-2-dipolyhydroxystearat (Handelsprodukt Dehymuls® PGPH) und Polyglyceryl-3-diisostearat (Handelsprodukt Lameform® TGI),
- Lineare und verzweigte C₈-C₃₀-Fettsäuren sowie deren Na-, K-, Ammonium-, Ca-, Mg- und Zn - Salze.

Insbesondere sind die erfindungsgemäß einsetzbaren Zusatzstoffe ausgewählt aus der Gruppe der Feuchthaltemittel, Vitamine, Antioxidantien, Desinfektionsmittel, entzündungshemmenden Mittel oder regenerativen Substanzen.

Bevorzugte Haut-Feuchthaltemittel sind Oligo- oder Monosaccharide. Als Monosaccharide sind z.B. Glucose, Fructose, Galactose, Arabinose, Ribose, Xylose, Lyxose, Allose, Altrose, Mannose, Gullose, Idose und Talose, die Desoxyzucker Fucose und Rhamnose sowie Aminozucker wie z.B. Glucosamin oder Galactosamin geeignet. Bevorzugt sind Glucose, Fructose, Galactose, Arabinose und Fucose; Glucose ist besonders bevorzugt.
Geeignete Oligosaccharide sind aus 2 bis 10 Monosaccharideinheiten zusammengesetzt, z.B. Saccharose, Lactose oder Trehalose. Ein besonders bevorzugtes Oligosaccharid ist Saccharose.

Unter Vitaminen sind hier neben den Vitaminen auch Pro-Vitamine und Vitaminvorstufen, insbesondere der Vitamine A (Retinol) und B zu verstehen. Von der Gruppe der Vitamin B-Komplexe sind besonders bevorzugt Vitamin B₃ (Nikotinsäure und Nikotinsäureamide) sowie Vitamin B₅ (Pantothensäure, Panthenol und seine Derivate). Erfindungsgemäß einsetzbare Derivate des Panthenols sind insbesondere die Ether und die Ester des Panthenols sowie kationisch derivatisierte Panthenole. Besonders bevorzugt werden das Panthenoltriacetat, der Panthenolmonoethylether sowie die Derivate des 2-Furanon unter Einschluss aller Stereoisomere (z.B. die im Handel erhältlichen Substanzen 4-Hydroxymethyl-γ-butyrolacton (Merck), 3,3-Dimethyl-2-hydroxy-γ-butyrolacton (Aldrich), und 2,5-Dihydro-5-methoxy-2-furanon (Merck)) eingesetzt. Ein erfindungsgemäß außerordentlich bevorzugtes Derivat des 2-Furanons ist Dihydro-3-hydroxy-4,4-dimethyl-2(3H)-Furanon, das sogenannte Pantolacton (Merck).

Als Antioxidantien geeignet sind Aminosäuren oder deren Derivate, Carotinoide, Carotine, Ascorbinsäure (Vitamin C), Tocopherole (Vitamin E), insbesondere α-Tocopherol sowie seine Ester, z.B. das Acetat, das Nicotinat, das Phosphat oder das Succinat, sowie Gallussäureester oder andere Gallussäurederivate. Besonders bevorzugt ist der Einsatz von Bisabolol und/oder Kamillenextrakten als entzündungshemmende Mittel.

Als Desinfektionsmittel eignen sich beispielsweise phenolhaltige, etherische Öle, quaternäre Ammoniumverbindungen (wie z.B. Cetrimoniumbromid oder Benzalkoniumchlorid), Chlorhexidindihydrochlorid,- diacetat, oder -digluconat.

Ein weiterer Gegenstand der Erfindung ist ein Verfahren zur Herstellung des erfindungsgemäßen Pflasters, dass dadurch gekennzeichnet ist, dass ein oder mehrere Polymere sowie mindestens ein Wirkstoff mit adstringierender und/oder blutstillender Wirkung in einem Wasser enthaltenden Lösungsmittel gelöst, emulgiert oder dispergiert werden, auf einen Träger aufgebracht werden, und dass anschließend das Lösungsmittel verdampft wird, wobei einer der adstringierenden und/oder blutstillenden Wirkstoffe ausgewählt ist aus Tanninen, Aluminium-, Zink-, Zirkonium-, Calcium- oder Bismutsalzen.

Das Lösungsmittel kann neben Wasser beispielsweise auch Anteile organischer, insbesondere leichtflüchtiger Lösungsmittel, wie z.B. Ether, Ethanol und/oder andere Alkohole enthalten.
Das Polymergemisch wird auf einen Träger aufgebracht und das Lösungsmittel anschließend verdampft. Die in Wasser löslichen, emulgierbaren oder dispergierbaren, aktiven Substanzen werden dadurch gleichmäßig verteilt in dem resultierenden Polymerfilm eingeschlossen.
In der Regel geschieht diese Trocknung an der Luft, es kann aber auch leichter Unterdruck angewendet werden, um den Trockenvorgang zu beschleunigen. Dabei ist darauf zu achten, dass der Unterdruck nicht so stark sein darf, dass sich im Polymerfilm zu große Blasen aufgrund der austretenden Gase entwickeln.

Gemäß einer besonderen Ausführungsform werden zur besseren Handhabbarkeit des Pflasters Träger mit dehäsiven Eigenschaften verwendet, weil sich das erfindungsgemäße Pflaster so sowohl im trockenen als auch im feuchten Zustand gut von diesem Träger ablösen lässt. Dabei kann es sich unter anderem, aber nicht ausschließlich, um beschichtete Träger, insbesondere beschichtetes Papier (z.B. silikoniertes Papier oder Wachspapier) handeln. Nicht wasserlösliche Folien (beispielsweise auch aus Metall) oder Polymerfilme (z.B. Teflonfolie) sind ebenfalls geeignet. Verschiedene Darreichungsformen des Pflasters mit und ohne Träger sind möglich.

Das erfindungsgemäße Pflaster kann insbesondere zur Blutstillung bei kleinen Wunden, wie Kratzern oder Schnitten, verwendet werden.
Häufig treten solche Schnitte im Gesicht bei der Nassrasur oder an den Händen bei Arbeiten mit scharfen Gegenständen wie beispielsweise Messern auf.

Ein Vorteil des erfindungsgemäßen Pflasters ist die schnelle Blutstillung, die von den adstringierenden Substanzen bewirkt wird.
Das erfindungsgemäße Pflaster ist daher besonders gut für Wunden ohne große Oberfläche mit einigermaßen glatten Rändern geeignet, die sonst nur schwer verheilen und z.T. stark bluten, da bei solchen Voraussetzungen die adstringierenden Eigenschaften des erfindungsgemäßen Pflasters ein optimales Zusammenziehen und Verschließen der Wunde bewirken.

Die folgenden Beispiele sollen die vorliegende Erfindung verdeutlichen, ohne sie hierauf zu beschränken.

### Beispiele:

### Beispiel 1:

20 g Polyvinylalkohol (Mowiol® 26-88, Clariant, Mᵣ= 160 000 g/mol ± 15%) wurden in 90 g Wasser unter Rühren in der Wärme gelöst. Zu der viskosen Flüssigkeit wurden 0,2 g Aluminiumchlorhydrat unter Rühren zugegeben. Die resultierende klare Lösung wurde danach auf einer Teflonfolie mit einem Glasstab ausgestrichen. Eine definierte Schichtdicke von 100 µm wurde dabei durch einen Rakel eingestellt. Das Lösungsmittel verdampfte innerhalb von 10 h durch Stehen an der Luft. Nach dem Trocknen wurden aus dem ausgehärteten Polymerfilm Pflaster in geeigneter Größe ausgestanzt. Die Teflonfolie diente bis zur Applikation als Träger für das Pflaster.

### Beispiel 2:

Analog zu Beispiel 1 wurde ein Polymerfilm aus 20 g Hydroxyethylcellulose (Natrosol® 250M, Aqualon, Mᵣ≈ 720 000 g/mol) mit 0,2 g Kalium-Aluminium-Sulfat hergestellt. Die Polymermasse wurde mit einer Schichtdicke von 250 µm ausgestrichen und luftgetrocknet.

10 Probanden wendeten das Produkt mehrfach in einem Zeitraum von zwei Wochen bei der täglichen Nassrasur zur Blutstillung an. Bei Auftreten einer Schnittwunde wurde das Pflaster auf die zusätzlich angefeuchtete Hautstelle gedrückt, wodurch es gut auf der Haut haftete. Die Blutung wurde innerhalb von zwei Minuten gestoppt. Trotz der guten Klebeeigenschaften ließ sich das Pflaster danach mühelos, ohne Rückstände und ohne Schmerzen wieder von der Haut abziehen, ohne die Wunde wieder aufzureißen. Bei der Befragung der Probanden beurteilten alle die blutstillenden Eigenschaften des Pflasters und die Hafteigenschaften als sehr gut. Die Hautverträglichkeit wurde ebenfalls von allen Probanden mit sehr gut oder gut bewertet.

## Patentansprüche

1. Blutstillendes Hautpflaster mit diskreter Form, **dadurch gekennzeichnet; dass** das Pflaster einen einschichtigen Polymerfilm mit mindestens. einem darin gelösten oder dispergierten, adstringierenden und/oder blutstillenden Wirkstoff umfasst, wobei einer der adstringierenden und/oder blutstillenden Wirkstoffe ausgewählt ist aus Tanninen, Aluminium-, Zink-, Zirkonium-, Calcium- oder Bismutsalzen.

2. Pflaster nach Anspruch 1, **dadurch gekennzeichnet, dass** der Polymerfilm ein oder mehrere Polymere umfasst.

3. Pflaster nach Anspruch 1 oder 2, **dadurch gekennzeichnet, dass** das Polymer ausgewählt ist aus der Klasse der Polyvinylalkohole, Polyvinylpyrrolidone, Polyvinylpyrrolidon-Vinylacetat-Copolymerisate, Polysaccharide, Polypeptide, Polyoxazoline, Homo- und Copolymerisate der Vinylphosphon-, Acryl- oder Methacrylsäure oder deren Derivate.

4. Pflaster nach einem der vorstehenden Ansprüche, **dadurch gekennzeichnet, dass** das Pflaster weitgehend transparent ist.

5. Pflaster nach einem der vorstehenden Ansprüche, **dadurch gekennzeichnet, dass** die Schichtdicke des Polymerfilms zwischen 0,001 und 1 mm und insbesondere zwischen 0,01 bis 0,25 mm liegt.

6. Pflaster nach einem der vorstehenden Ansprüche, **dadurch gekennzeichnet, dass** der Polymerfilm einen Feuchtigkeitsgehalt von 0 bis 50 Gew.-%, insbesondere 10 bis 35 Gew.-% aufweist.

7. Pflaster nach einem der vorstehenden Ansprüche, **dadurch gekennzeichnet, dass** die Aluminiumsalze ausgewählt sind aus der Gruppe von Aluminium-Hydroxychloriden, Aluminiumderivaten von Allantoin, Kalium-Aluminiumsulfat, Ammonium-Aluminiumsulfat, Aluminium-Zirkonium-Hydroxychloride oder Aluminium-Zirkonium-Hydroxychlorid-Glycin-Komplex.

8. Pflaster nach einem der vorstehenden Ansprüche, **dadurch gekennzeichnet, dass** der Polymerfilm Glycerin, Sorbitol und/oder andere Substanzen enthält, die den Polymerfilm geschmeidiger und/oder flexibler machen.

9. Pflaster nach einem der vorstehenden Ansprüche, **dadurch gekennzeichnet, dass** ein oder mehrere Wirk-, Pflege- oder Hilfsstoffe enthalten sind, insbesondere ausgewählt aus der Gruppe der Feuchthaltemittel, Vitamine, Antioxidantien, Desinfektionsmittel, regenerativen Substanzen oder entzündungshemmenden Mittel.

10. Pflaster nach Anspruch 9, **dadurch gekennzeichnet, dass** es Monosaccharide, Oligosaccharide, Panthenol, Pantothensäure, Pantolacton, Derivate von 2-Furanon, Chlorhexidinderivate, Allantoin, Aloe Vera und/oder Bisabolol enthält.

11. Pflaster nach einem der Ansprüche 1 bis 10 zur Wundversorgung, insbesondere bei Schnittverletzungen und kleinen Wunden.

12. Verfahren zur Herstellung eines Pflasters nach einem der Ansprüche 1 bis 11, **dadurch gekennzeichnet, dass** ein oder mehrere Polymere sowie mindestens ein Wirkstoff mit adstringierender und/oder blutstillender Wirkung in einem Wasser enthaltenden Lösungsmittel gelöst, emulgiert oder dispergiert werden, auf einen Träger aufgebracht werden, und dass anschliessend das Lösungsmittel verdampft wird, wobei einer der adstringierenden und/oder blutstillenden Wirkstoffe ausgewählt ist aus Tanninen, Aluminium-, Zink-, Zirkonium-, Calcium- oder Bismutsalzen.

13. Verfahren zur Herstellung eines Pflasters nach Anspruch 12, **dadurch gekennzeichnet, dass** der Träger dehäsive Eigenschaften aufweist.

## Claims

1. A blood-staunching skin plaster with a discrete form, **characterized in that** the plaster comprises a single-layer polymer film with at least one astringent and/or blood-staunching active component dissolved or dispersed therein, one of the astringent and/or blood-staunching active components being selected from tannins, aluminium, zinc, zirconium, calcium or bismuth salts.

2. A plaster as claimed in claim 1, **characterized in that** the polymer film comprises one or more polymers.

3. A plaster as claimed in claim 1 or 2, **characterized in that** the polymer is selected from the class of polyvinyl alcohols, polyvinyl pyrrolidones, polyvinyl pyrrolidone/vinyl acetate copolymers, polysaccharides, polypeptides, polyoxazolines, homo- and copolymers of vinyl phosphonic acid, acrylic acid or methacrylic acid or derivatives thereof.

4. A plaster as claimed in any of the preceding claims, **characterized in that** it is largely transparent.

5. A plaster as claimed in any of the preceding claims, **characterized in that** the layer thickness of the polymer film is between 0.001 and 1 mm and more particularly between 0.01 and 0.25 mm.

6. A plaster as claimed in any of the preceding claims, **characterized in that** the polymer film has a moisture content of 0 to 50% by weight and, more particularly, 10 to 35% by weight.

7. A plaster as claimed in any of the preceding claims, **characterized in that** the aluminium salts are selected from the group of aluminium hydroxychlorides, aluminium derivatives of allantoin, potassium aluminium sulfate, ammonium aluminium sulfate, aluminium zirconium hydroxychlorides or aluminium zirconium hydroxychloride/glycine complex.

8. A plaster as claimed in any of the preceding claims, **characterized in that** the polymer film contains glycerol, sorbitol and/or other substances which make the polymer film more pliable and/or flexible.

9. A plaster as claimed in any of the preceding claims, **characterized in that** it contains one or more active components, care components or auxiliaries, more particularly selected from the group of humectants, vitamins, antioxidants, disinfectants, regenerative substances or antiinflammatory agents.

10. A plaster as claimed in claim 9, **characterized in that** it contains monosaccharides, oligosaccharides, panthenol, pantothenic acid, pantolactone, derivatives of 2-furanone, chlorhexidine derivatives, allantoin, aloe vera and/or bisabolol.

11. A plaster as claimed in any of claims 1 to 10 for wound care, more particularly for cuts and minor wounds.

12. A process for the production of the plaster claimed in any of claims 1 to 11, **characterized in that** one or more polymers and at least one astringent and/or blood-staunching active component are dissolved, emulsified or dispersed in a water-containing solvent and applied in this form to a carrier and the solvent is evaporated, one of the astringent and/or blood-staunching active components being selected from tannins, aluminium, zinc, zirconium, calcium or bismuth salts.

13. A process as claimed in claim 12 for the production of a plaster, **characterized in that** the carrier has dehesive properties.

## Revendications

1. Pansement cutané hémostatique de forme discrète, **caractérisé en ce que** le pansement comprend un film polymère monocouche contenant au moins un principe actif astringent et/ou hémostatique dissous ou dispersé dans le film, l'un des principes actifs astringents et/ou hémostatiques étant choisi parmi les tannins, les sels d'aluminium, de zinc, de zirconium, de calcium ou de bismuth.

2. Pansement selon la revendication 1, **caractérisé en ce que** le film polymère comprend un ou plusieurs polymères.

3. Pansement selon l'une des revendications 1 ou 2, **caractérisé en ce que** le polymère est choisi dans la classe comprenant l'alcool polyvinylique, la polyvinylpyrrolidone, les copolymères polyvinylpyrrolidone-acétate de vinyle, les polysaccharides, les polypeptides, les polyoxazolines, les homo- et copolymères de l'acide vinylphosphonique, acrylique ou méthacrylique ou de leurs dérivés.

4. Pansement selon l'une quelconque des revendications précédentes, **caractérisé en ce que** le pansement est transparent dans une grande mesure.

5. Pansement selon l'une quelconque des revendications précédentes, **caractérisé en ce que** l'épaisseur de couche du film polymère est comprise entre 0,001 et 1 mm et en particulier, entre 0,01 et 0,25 mm.

6. Pansement selon l'une quelconque des revendications précédentes, **caractérisé en ce que** le film polymère présente une teneur en humidité de 0 à 50 % en poids, en particulier de 10 à 35 % en poids.

7. Pansement selon l'une quelconque des revendications précédentes, **caractérisé en ce que** les sels d'aluminium sont choisis parmi les hydroxychlorures d'aluminium, les dérivés aluminiques de l'allantoïne, le sulfate d'aluminium et de potassium, le sulfate d'aluminium et d'ammonium, l'hydroxychlorure d'aluminium et de zirconium ou le complexe hydroxychlorure d'aluminium et de zirconium - glycine.

8. Pansement selon l'une quelconque des revendications précédentes, **caractérisé en ce que** le film polymère contient du glycérol, du sorbitol et/ou d'autres substances qui rendent le film polymère plus malléable et/ou plus souple.

9. Pansement selon l'une quelconque des revendications précédentes, **caractérisé en ce qu'**il contient un ou plusieurs principes actifs, substances de soin ou adjuvants, choisis en particulier parmi les agents de maintien de l'humidité, les vitamines, les antioxydants, les désinfectants, les substances régénératrices ou les anti-inflammatoires.

10. Pansement selon la revendication 9, **caractérisé en ce qu'**il contient des monosaccharides, des oligosaccharides, du panthénol, de l'acide panthoténique, de la pantolactone, des dérivés de la 2-furanone, des dérivés de la chlorhexidine, de l'allantoïne, de l'aloe vera et/ou du bisabolol.

11. Pansement selon l'une quelconque des revendications 1 à 10, destiné à panser des plaies, en particulier dans le cas d'entailles et de petites blessures.

12. Procédé de fabrication d'un pansement selon l'une quelconque des revendications 1 à 11, **caractérisé en ce que** l'on dissout, émulsifie ou disperse dans un solvant contenant de l'eau un ou plusieurs polymères ainsi qu'au moins un principe actif ayant une action astringente et/ou hémostatique, que l'on dispose la solution, émulsion ou dispersion sur un support et **en ce que** l'on évapore ensuite le solvant, un des principes actifs astringents et/ou hémostatiques étant choisi parmi les tannins, les sels d'aluminium, de zinc, de zirconium, de calcium ou de bismuth.

13. Procédé de fabrication d'un pansement selon la revendication 12, **caractérisé en ce que** le support présente des propriétés déhésives.
